(19) Europäisches Patentamt
European Patent Office
Office européen des brevets

(11) EP 4 397 331 B1

(12) EUROPEAN PATENT SPECIFICATION

(45) Date of publication and mention of the grant of the patent:
**04.02.2026 Bulletin 2026/06**

(21) Application number: **23213147.4**

(22) Date of filing: **29.11.2023**

(51) International Patent Classification (IPC):
***A61L 27/48*** *(2006.01)* ***A61L 27/52*** *(2006.01)*
***A61L 27/58*** *(2006.01)*

(52) Cooperative Patent Classification (CPC):
(C-Sets available)
**A61L 27/52; A61L 27/48; A61L 27/58** (Cont.)

(54) **CROSS-LINKABLE POLYMERS FOR FABRICATION OF BIODEGRADABLE 3D HYDROGEL SCAFFOLD, AND PROCESS THEREOF**

VERNETZBARE POLYMERE ZUR HERSTELLUNG EINES BIOLOGISCH ABBAUBAREN 3D-HYDROGELGERÜSTS UND VERFAHREN DAFÜR

POLYMÈRES RÉTICULABLES POUR LA FABRICATION D'UN ÉCHAFAUDAGE BIODÉGRADABLE EN HYDROGEL 3D ET PROCÉDÉ ASSOCIÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.01.2023 IN 202221063505**

(43) Date of publication of application:
**10.07.2024 Bulletin 2024/28**

(73) Proprietor: **Indian Oil Corporation Limited**
**Mumbai, Maharashtra 400 051 (IN)**

(72) Inventors:
- **GAJENDRAN, Mani**
  **121007 Faridabad (IN)**
- **OTA, Jyotiranjan**
  **121007 Faridabad (IN)**
- **HAIT, Samik Kumar**
  **121007 Faridabad (IN)**
- **RAMAKUMAR, Sankara Sri Venkata**
  **121007 Faridabad (IN)**

(74) Representative: **Turner, Craig Robert et al**
**Venner Shipley LLP**
**200 Aldersgate**
**London EC1A 4HD (GB)**

(56) References cited:
**CN-A- 108 102 112 US-A1- 2021 346 576**

- **ASHKAN TAVAKOLI NAEINI: "Poly(citric acid)-block-poly(ethylene glycol) copolymers-new biocompatible hybrid materials for nanomedicine", NANOMEDICINE: NANOTECHNOLOGY, BIOLOGY, AND MEDICINE, vol. 6, no. 4, 13 January 2010 (2010-01-13), AMSTERDAM, NL, pages 556 - 562, XP093161403, ISSN: 1549-9634, DOI: 10.1016/j.nano.2009.11.008**
- **YING WANG: "Photo-crosslinked biodegradable elastomers for controlled nitric oxide delivery", BIOMATERIALS SCIENCE, vol. 1, no. 6, 1 January 2013 (2013-01-01), GB, pages 625, XP093161431, ISSN: 2047-4830, DOI: 10.1039/c3bm00169e**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/48, C08L 89/06, C08L 71/02**

## Description

### FIELD OF THE INVENTION

The present invention relates to a biodegradable 3D composite hydrogel scaffold comprising a cross-linkable synthetic biodegradable polymer methacrylated poly multi-carboxylic acid-polyethylene glycol (MA-PMCA-PEG) and a natural polymer maleated gelatin (GEL-MEA). More particularly, the present invention relates to a simple and cost-effective development of cross-linkable synthetic and natural polymers for the fabrication of 3D composite biodegradable hydrogels that exhibits anti-viral properties and can be utilized in disinfectant spray or as a coating matrix for face mask. The present invention deals with production of new cross-linkable polymers and a process to achieve biodegradable hydrogels. A multicarboxylic acid-b-polyethylene glycol (PMCA-PEG), synthesized by a direct melt polycondensation technique is methacrylated using glycidyl methacrylate to prepare cross-linkable methacrylated PMCA-PEG (MA-PMCA-PEG), which is a new biodegradable material with excellent cross-linking properties. The natural polymer gelatin (GEL) is reacted with maleic anhydride (MEA) to get cross-linkable maleated gelatin (GEL-MEA). The approach of using maleated gelatin (GEL-MEA) as a crosslinkable polymer stages a number of advantages over the conventional thiolated cross-linkable polymers. The synthetic polymer MA-PMCA-PEG and modified natural polymer GEL-MEA were cross-linked by using ammonium persulfate and tetramethylethylenediamine (TEMED) to get a distinct 3D hydrogel scaffold. The hydrogel scaffold could be prepared in nano or micro size via water-in-oil (W/O) emulsion method. The nanohydrogel based platform could be used as a carrier for anti-viral nanoparticles in disinfectant spray or as a coating matrix for face mask.

### BACKGROUND OF THE INVENTION

Hydrogel scaffolds used for biomedical applications are usually fabricated by cross-linking functional polymer materials using a cross-linker and initiator. Synthesis of most of the reported cross-linkable polymers involves expensive materials, tedious process, carcinogenic chemicals or non-degradable polymers. The poly (ethylene glycol) diacrylate (PEGDA) has been used as a cross- linkable polymer to fabricate hydrogel scaffolds for soft tissue regeneration (Jeremy, WO, 2006/004951 A2) and drug delivery application (Wu *et al.,* 2010, US7670616B2). The PEGDA based hydrogels are non cell adhesive; hence biomaterials such as cross linkable collagen, hyaluronic acid, and GEL have been cross-linked with PEGDA to get cell adhesive biocompatible hydrogel. The PEGDA is a non-degradable polymer and very expensive, hence, scaling up of the hydrogel formulation using PEGDA in industrial scale is not viable.

Recently, citric acid-PEG based copolymers received great attention in biomedical field. For instance, Naeini et al., have reported the synthesis and self-assembly of poly(citric acid) (PCA)-block-poly(ethylene glycol) (PEG) copolymer [Nanomedicine: Nanotechnology, Biology, and Medicine 6 (2010) 556-562]. Then, similar citric acid-PEG based polymers were synthesized and used for drug delivery and nanomedicine applications. Naeini et al., have used the PCA-PEG-PCA polymer to synthesize necklace shaped gold NPs [European Polymer Journal 46 (2010) 165-170]. Gajendiran et al., have reported the synthesis and selfaggregation induced emission property of citric acid-PEG hyper branched polymer [Scientific Reports volume 7, Article number: 16418 (2017)], and the same research group has used the citric acid-PEG copolymer for the green synthesis of gold nanoparticles [Journal Material Chemistry B, 2014, 2, 418-427]. However, the cross-linkable methacrylated citric acid-PEG polymer and further cross-linking of the polymer with other natural polymers have not been reported in literature.

Also, several modifications on gelatin with various functional moieties have been performed to make hydrogel scaffold. For instance, Kim *et al.,* have reported hydroxyl phenyl propionic acid modified GEL to get gelatin hydrogel via an enzymatic cross linking (Kim et al., ACS Appl. Bio Mater. 2020, 3, 1646-1655). Kurisawa *et al.,* have disclosed synthesis of hydroxylphenylpropionic acid (HPA) attached gelatin (GEL-HPA) via 1-Ethyl-3-(3-dimethylaminopropyl)carbodiimide (EDC) coupling reaction (Kurisawa *et al.,* WO 2019/226120 A1). Park *et al.,* have disclosed the preparation of hydroxylphenylacetic acid attached gelatin (GHPA) and formation of hydrogel via horseradish peroxidase (HRP) enzymatic cross-linking reaction (Park *et al.,* EP 2 448 610 B1). The GEL-HPA hydrogel was prepared by using an enzyme HRP as a cross-linker and hydrogen peroxide as a radical initiator. Bulcke *et al.,* have reported methacrylate modified GEL by reacting with methacrylic anhydride to prepare GEL hydrogel via photo cross-linking reaction (Bulcke et al., Biomacromolecules, 2000, 1, 31-38). Munoz *et al.,* have synthesized norbornene attached GEL to prepare GEL hydrogel via an orthogonal thiol-norbornene photochemistry (Monuz et al., Biomater. Sci., 2014, 2, 1063-1072). Bishop *et al.,* have disclosed the cross-linking of GEL using various external cross-linkers such as (1-ethyl-3-3-(dimethylamino)propyl carbodiimide) (EDC) and calcium chloride, but the cross-linking processes were time consuming processes (2 h) (Bishop *et al.,* US Pat. 5834232).

Thiolation of gelatin is a widely followed strategy to prepare cross-linkable gelatin (Stanley *et al.,* U.S. Pat. Appl. Publ. 2014, US 20140315805 A1 20141023). The thiolated gelatin could be cross-linked with PEGDA to prepare 3D hydrogel scaffold for biomedical application (Sungjun et al., Tissue Eng. Regen. Med. 2022, 19, 309-319).

However, the thiolated gelatin is highly reactive and involves in self cross-linking in an uncontrolled manner under atmospheric air. Hence, the shelf life of the product is a great hindrance for wide commercial use as it may not be useful for making hydrogel after exposure to atmospheric air. Hence, production of the thiolated gelatin based hydrogel in industrial scale is a difficult task. A new breed of material devoid of thiolated gelatin would be helpful to overcome this technical difficulty without compromising the gelation property.

Several cross-linkable polymers have been reported in literature for the fabrication of hydrogel scaffolds. Usually, the PEGDA, thiolated gelatin, thiolated alginate, thiolated chitosan, thiol modified heparin and hyaluronan have been used as cross-linkable polymers. The PEGDA is a non degradable polymer and the thiolating agents are highly toxic (Category II) in nature, and hence they are not environmentally benign materials. The thiolated polymers are highly expensive and hence, a huge investment is required for their production in large scale. Selfstability of the widely used thiolated polymers under atmospheric air is very low due to the oxidation of sulfhydryl group to form disulfide bond, and hence handling the thiolated polymers in industrial scale is not easy. Hence, new types of biodegradable advanced materials need to be developed via cost effective and simple synthetic procedures for the fabrication of hydrogel scaffold in large scale.

Also, synthesis of cross-linkable polymers such as thiolated gelatin, collagen involves expensive reagents such as Traut's reagent and $\gamma$-thiobutyrolactone. In addition, after synthesis, the cross-linkable polymers need to be purified by dialysis and followed by freeze drying. The dialysis tube is very expensive to purify the polymers in bulk scale. Hence, synthesis of the conventional cross-linkable polymers in bulk scale is expensive and for commercial application requires higher cost of investment.

Additionally, synthesis of cross-linkable gelatin involves a series of synthetic process steps Vis. i) addition of the thiolating agents to gelatin backbone under inert atmosphere, ii) dialysis and iii) freeze drying. Particularly, in a synthetic process of 1 g scale of thiolated gelatin, the freeze dryer should run for 4 to 5 days continuously to dry the sample completely. Hence, synthesis of the thiolated gelatin is a tedious process to scale up in bulk scale.

Hence, a simple and cost-effective synthetic procedure needs to be developed to synthesize the cross-linkable gelatin in bulk scale for commercial application.

Recently, the maleate moiety has been used as a cross-linking group to fabricate hydrogel. For instance, Sukhlaaied *et al.,* have reported the maleated polyvinyl alcohol and cross-linked with gelatin to prepare the graft polymer hydrogel [Sukhlaaied et al., Polym. Bull. 8, 2016, DOI 10.1007/s00289-016-1609-3]. Ali *et. al.* has directly polymerized the maleic anhydride on gelatin backbone in the presence of drug molecule to prepare a drug copolymer [Chemistry and Materials Research., Vol.7, No.5, 2015].

Moreover, in the reported papers, maleic anhydride was directly polymerized on gelatin, but the -C=C- of maleate molecule was not free for further cross linking. The cross-linkable maleated gelatin with free -C=C- on maleate moiety has not been reported in literature.

CN 108 102 112 relates to synthesis of an (N-maleyl gelatin) (N-MAGEL) aqueous solution by using gelatin and maleic anhydride. The product is cured, freeze dried and N-MAGEL aqueous solution is obtained.

US 2021/346576 relates to methods, compositions, devices, and systems for the 3D printing of biomedical implants. In particular, methods and systems are provided for 3D printing of biomedical devices (e.g., endovascular stents) using photo-curable biomaterial inks (e.g., methacrylated poly(diol citrate)).

## SUMMARY OF THE INVENTION

Accordingly, the present invention provides a biodegradable and environment friendly crosslinkable synthetic polymer methacrylated poly multi-carboxylic acid-polyethylene glycol (MA-PMCA-PEG) and a process for synthesising/-preparing the same by simple synthetic methods. The cross-linkable synthetic polymer MA-PMCA-PEG exhibits multiple active sites which could be used for cross-linking with the natural polymers such as gelatin, collagen and chitosan to get three-dimensional hydrogel scaffolds.

In addition, the cost effectively scalable MA-PMCA-PEG polymer could provide both the cross-linking moiety and free carboxylic acid group into the hydrogel network. The cross-linking moiety could be used to fabricate hydrogel scaffold, while the free carboxylic acid group could exhibit anti-viral property. Hence, the polymer could be used to get an ionic hydrogel matrix for antiviral application.

Described but not claimed is a simple and new and cost-effective process for the development of stable cross-linkable natural polymer maleated gelatin (GEL-MEA) having free -C=C- group for controlled cross-linking, via an in-house developed procedure using less expensive materials such as gelatin, maleic anhydride, water and acetone.

Fabrication of carboxylic acid enriched hybrid composite hydrogel by cross-linking the MA-PMCA-PEG polymer with other cross-linkable natural polymers such as GEL-MEA, which is new of its kind. The cross-linkable polymers could be used to fabricate anti-viral composite hydrogel in industrial scale by a cost-effective approach. The mechanical properties of the fabricated hydrogel could be tuned by varying the composition of MA-PMCA-PEG and GEL-MEA in the hydrogel scaffold.

The present invention provides a biodegradable 3D composite hydrogel scaffold comprising (i) a synthetic

biodegradable polymer methacrylated poly multi-carboxylic acid-polyethylene glycol (MA-PMCA-PEG) and (ii) a natural polymer maleated gelatin (GEL-MEA). The present invention also provides a process for the development of biodegradable 3D composite hydrogel comprising (i) Cross-linkable biodegradable and environment friendly synthetic polymer, i.e., MA-PMCA-PEG and (ii) Cross-linkable natural polymer, i.e., GEL-MEA for the fabrication of hydrogel scaffold at large scale. The new biodegradable MA-PMCA-PEG synthetic polymer is developed via cost effective and simple synthetic procedures. The crosslinkable natural polymer, GEL-MEA is synthesized by a simple and controlled synthetic procedure and is highly stable under normal atmospheric condition at room temperature for a minimum period of four months. Composite 3D hydrogel scaffolds are fabricated by cross-linking MA-PMCA-PEG and GEL-MEA. The synthesized polymer exhibits both cross-linking moiety and free carboxylic acid group.

The present invention describes two types of cross-linkable polymers: (i) Cross-linkable synthetic polymer, i.e. MA-PMCA-PEG and (ii) Cross-linkable natural polymer, i.e. GEL-MEA.

The cross-linkable synthetic polymer MA-PMCA-PEG is biodegradable and hence it is environmentally friendly.

The cross-linkable natural polymer, GEL-MEA was synthesized by a simple synthetic procedure. A mixture of aqueous and organic solvents was used in the synthesis of GEL-MEA, and the polymer product was recovered just by increasing the organic composition in the reaction mixture. The organic solvent could be recycled by rotary evaporation. Hence, the synthesis procedure for getting GEL-MEA is technically and economically important. The GEL-MEA described in the present disclosure is highly stable under normal atmospheric condition at room temperature for a minimum period of four months, and the cross-linking reaction with GEL-MEA could be carried by a controlled process.

## OBJECTIVES OF THE PRESENT INVENTION

It is the primary objective of the present invention to develop a biodegradable 3D composite hydrogel by cross-linking synthetic and natural polymer.

It is one of the main objectives of the present invention to synthesize the synthetic cross-linkable polymers in cost effective manner with less expensive materials.

It is one of the main objectives of the present invention to synthesize the synthetic cross-linkable polymers in simple, controlled and scalable manner.

It is yet another objective of the present invention to synthesize the polymer with both cross-linking moiety and free carboxylic acid group for anti-viral property.

## BRIEF DESCRIPTION OF THE DRAWINGS:

**Figure 1:** Illustrates synthesis of cross-linkable MA-PMCA-PEG.
**Figure 2:** Illustrates synthesis of cross-linkable GEL-MEA.
**Figure 3:** Illustrates process for scaling up of MA-PMCA-PEG polymer.
**Figure 4:** Illustrates fabrication of 3D hydrogel matrix using the cross-linkable polymers MA-PMCA-PEG and GEL-MEA.
**Figure 5:** Illustrates FTIR spectra of (a) PMCA-PEG and (b) MA-PMCA-PEG polymers.
**Figure 6:** Illustrates $^1$H-NMR spectra of (a) PMCA-PEG and (b) MA-PMCA-PEG recorded in $D_2O$.
**Figure 7:** Illustrates $^{13}$C-NMR spectra of (a) PPMCA-PEG and (b) MA-PMCA-PEG recorded in $D_2O$.
**Figure 8:** Illustrates $^1$H-NMR spectra of (a) gelatin and (b) GEL-MEA recorded in $D_2O$.
**Figure 9:** Illustrates $^{13}$C-NMR spectra of (e) gelatin and (b) GEL-MEA recorded in $D_2O$.
**Figure 10:** Illustrates DSC thermograms of (a) PMCA-PEG and (b) MA-PMCA-PEG.
**Figure 11:** Illustrates (A) Stress Vs Strain plot of compression test and (B) compressive modulus of different hydrogel matrices. HG1, HG2 and HG3 exhibit 0.5, 0.33 and 0.0 ratio of MA-PMCA-PEG/GEL-MEA.

## ABBREVIATIONS

PEG: polyethylene glycol
MA: maleic anhydride
PMCA: poly multi-citric acid/ poly multi-carboxylic acid.
GEL: gelatin
MEA: methacrylated
TEMED: tetramethylethylenediamine

## DETAILED DESCRIPTION OF THE INVENTION

For the purpose of promoting an understanding of the principles of the invention, reference will now be made to the embodiments in the specific language will be used to describe the same. Unless otherwise defined, all technical and scientific terms used herein have the same meaning as commonly understood by one of ordinary skilled in the art to which this invention belongs. The composition, methods, and examples provided herein are illustrative only and not intended to be limiting.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described.

The terminology and structure employed herein is for describing, teaching, and illuminating some embodiments and their specific features and elements and does not limit, restrict, or reduce the scope of the invention.

The present invention provides first time a synthesis of methacrylated poly citric acid-b-poly ethylene glycol branched polymer (MA-PMCA-PEG), a new biodegradable material. The MA-PMCA-PEG polymer exhibits both cross-linkable moiety and free carboxylic acid group. Hence, the polymer could be used to get an ionic hydrogel matrix for antiviral application.

The present disclosure also describes a synthesis of cross-linkable GEL-MEA for the first time by a new procedure, the GEL-MEA is highly stable under atmospheric air, and cross-linking reaction of GEL-MEA could be carried in a controlled manner.

Fabrication of carboxylic acid enriched hybrid composite hydrogel by cross-linking the MA-PMCA-PEG polymer with other cross-linkable natural polymers such as GEL-MEA, which is new of its kind.

Accordingly, the present invention provides a biodegradable 3D composite hydrogel scaffold comprising:

(i) a synthetic biodegradable polymer methacrylated poly multi-carboxylic acid-polyethylene glycol (MA-PMCA-PEG) and
(ii) a modified natural polymer maleated gelatin (GEL-MEA).

In one of the features of the present invention, the biodegradable 3D composite hydrogel scaffold has a ratio of MA-PMCA-PEG/GEL-MEA varies a range of 0.33 - 0.5.

The present invention provides a process for fabricating a biodegradable 3D composite hydrogel scaffold comprising:

(i) mixing of predetermined volume of a solution of MA-PMCA-PEG and a solution of GEL-MEA to obtain a polymer solution, wherein different hydrogels are obtained by varying the concentration ratio of MA-PMCA-PEG/GEL-MEA; and
(ii) preparing hydrogel from the polymer solution and molding the hydrogel to obtain the biodegradable 3D composite hydrogel scaffold.

In one of the features, the present invention provides a process for fabricating a biodegradable 3D composite hydrogel scaffold comprising:

(i) mixing of predetermined volume of a solution of MA-PMCA-PEG and a solution of GEL-MEA to obtain a polymer solution, wherein different hydrogels are obtained by varying the concentration ratio of MA-PMCA-PEG/GEL-MEA;
(ii) adding tetramethylethylenediamine (TEMED) to the polymer solution, followed by addition of a solution of ammonium persulfate (APS) and incubating till the formation of hydrogel, and
(iii) molding the hydrogel to a disc shaped 3D matrix using polypropylene mold tool to obtain the biodegradable 3D composite hydrogel scaffold.

In one of the features of the present invention, the concentration of MA-PMCA-PEG is in the range of 40-60 %, w/v. The concentration of GEL-MEA is in the range of 40-60 %, w/v. The solution of ammonium persulfate is in the range of

5-15%, w/v. In another feature of the present invention, the concentration of MA-PMCA-PEG is in the range of 40-60 %, w/v, GEL-MEA is in the range of 40-60 %, w/v, and solution of ammonium persulfate is in the range of 5-15%, w/v are prepared in deaerated water.

In another feature of the present invention, the volume of MA-PMCA-PEG and GEL-MEA is in the range of 300-900 μL, the volume of TEMED is in the range of 50-70 μL, the volume of ammonium persulphate is in the range of 40-50 μL.

In yet another feature of the present invention, the incubation is carried out at a temperature in the range of 30-40 °C for 10-30 min till the formation of hydrogel.

In yet another feature of the present invention, the composite 3D hydrogel with 0.50 and 0.33 ratio of MA-PMCA-PEG/GEL-MEA exhibit swelling factor of 8.0 and 6.2 respectively.

In yet another feature of the present invention, the compression modulus of the composite 3D hydrogel with 0.50 and 0.33 ratio of MA-PMCA-PEG/GEL-MEA is 35.1 and 19.3 kPa respectively.

Described but not claimed is a synthetic biodegradable cross linkable polymer: methacrylated poly multi-carboxylic acid-polyethylene glycol (MA-PMCA-PEG)

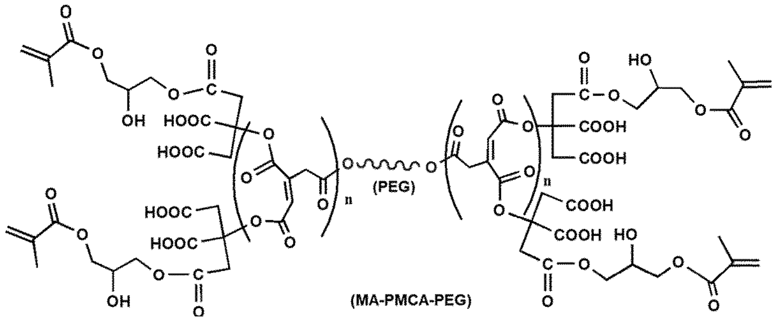

(MA-PMCA-PEG)

The present invention provides a process for preparation of methacrylated poly multicarboxylic acid-polyethylene glycol (MA-PMCA-PEG) comprising reacting poly multicarboxylic acid-polyethylene glycol (PMCA-PEG) polymer in dimethylformamide (DMF) with glycidyl methacrylate (GMA) in the presence of triethylamine (TEA) and adding diethyl ether as a non-solvent to recover MA-PMCA-PEG.

In the process for preparation of MA-PMCA-PEG, the amount of GMA is in the range of 0.02-0.1 mol, and the amount of triethylamine is in the range of 5-15 mL.

In another feature of the present invention, in the process for preparing MA-PMCA-PEG, the reaction is carried out at a temperature in the range of 60-80 °C for 15 to 24 h under dark and inert atmosphere with strong agitation.

Described but not claimed is a process for preparing maleated gelatin (GEL-MEA) comprising:
dissolving gelatin in water to obtain a gelatin solution, and reacting the gelatin solution with maleating agent in acetone to obtain the cross linkable GEL-MEA.

The maleating agent is maleic anhydride; and the amount of gelatin in water is in the range of 5-15 %, w/v and the amount of maleic anhydride in acetone is in the range of 40-60 %, w/v. In another feature of the present invention, water is de-ionized distilled water (DDW).

In the process for preparing GEL-MEA, the reaction is carried out at a temperature in the range of 40-60 °C for 4 h.

The present invention describes the development of cross-linkable polymers suitable for fabrication of hydrogel scaffolds. Hydrogel scaffolds are usually fabricated by cross-linking functional polymer materials using a cross-linker and initiator. In the present study, the cross-linking moieties such as maleate or methacrylate groups have been attached on the natural polymer gelatin or synthetic polymer PMCA-PEG. The cross-linkable natural or synthetic polymers have been synthesized by simple and easily scalable techniques. The present disclosure provides an alternative cross-linkable gelatin for thiolated gelatin to increase its selfstability under normal atmospheric conditions. The cross-linkable polymers were cross-linked to obtain hydrogel scaffold.

In one embodiment, the present invention provides a synthesis of polymer PMCA-PEG by direct melt reaction of citric acid and PEG (Mw = 400 - 2000 g/mol) using stannous chloride dihydrate as a catalyst. After the reaction, the polymer was dissolved in dimethyl formamide (DMF).

In another embodiment, methacrylation of the polymer PMCA-PEG in DMF by using glycidyl methacrylate and triethylamine is described. After the reaction, the polymer product MA-PMCA-PEG was recovered by the addition of diethyl ether, and dried under rotary evaporation. In another embodiment the invention provides that the polymer MA-PMCA-PEG is easily scalable in a cost-economic manner using custom designed reactor.

Additionally, maleation of the natural polymer gelatin using maleic anhydride is described. For maleation of gelatin, a mixture of acetone/water system was used as solvent. After the reaction, the GEL-MEA was precipitated by addition of excess acetone, and at the same time the unreacted maleic anhydride was washed out in acetone. Then, the

GEL-MEA was dried by rotary evaporation. The GEL-MEA was synthesized with cost-economic reagents, and it is highly stable under room temperature and atmospheric air.

In a preferred embodiment, fabrication of composite 3D hydrogel by cross-linking MA-PMCA-PEG and GEL-MEA is described. The hydrogel scaffold was fabricated to a disc shaped 3D matrix using polypropylene mold tool. The mechanical property and swelling factor of the fabricated hydrogel are disclosed. The mechanical property of the 3D composite hydrogels could be altered by changing chemical composition of MA-PMCA-PEG and GEL-MEA in the hydrogel matrix.

The present invention involves synthesis of cross-linkable synthetic and natural polymers by simple synthetic methods. The PEGDA has been widely used to make hydrogel scaffolds with natural polymers. However, the high molecular weight PEGDA is a non-degradable polymer. The linear PEGDA contains only two active sites for cross-linking, and hence the reactivity will be lesser for cross-linking. In this present invention, the cross-linkable synthetic polymer MA-PMCA-PEG exhibits multiple active sites which could be used for cross-linking with the natural polymers such as gelatin, collagen and chitosan to get three dimensional hydrogel scaffolds. Since, the MA-PMCA-PEG is polyester, it is biodegradable and environmental friendly to use. The MA-PMCA-PEG cross-linkable polymer is scalable in a cost-economic manner. In addition, MA-PMCA-PEG polymer could provide both cross-linking moiety and free carboxylic acid group into the hydrogel network. The cross-linking moiety could be used to fabricate hydrogel scaffold, while the free carboxylic acid group could exhibit anti-viral property.

This present disclosure also describes the development of cross-linkable natural polymer gelatin. In the reported works, the gelatin was widely modified with thiol groups to make hydrogel scaffold. Though the thiol based synthetic pathway of making hydrogel scaffold is a well-known and fruitful method, the commonly used thiolating agent 2-iminothiolane hydrochloride (Traut's reagent) is very expensive and hence production of bulk scale thiolated polymers is challenging. In addition, the conventional thiolated polymers are oxidized under normal atmospheric condition, involve in self cross-linking in uncontrolled manner and hence, fabrication of shape controlled hydrogel scaffold using the oxidized thiolated gelatin could not be achieved under normal atmospheric storage condition. In the present study a simple and new strategy has been disclosed to prepare cross-linkable gelatin which is highly stable under atmospheric air without undergoing self cross-linking.

The GEL-MEA described herein has been synthesized in a simple and cost effective method. The less expensive materials such as gelatin, maleic anhydride, water and acetone are only used in the present invention to synthesize GEL-MEA. Additionally, the synthesized GEL-MEA is highly stable under normal atmospheric air condition, and the cross-linking reaction using this polymer could be carried by a controlled approach. Hence, this present disclosure describes simple and cost effective methods for the development of crosslinkable synthetic and natural polymers. The cross-linkable polymers could be used to fabricate anti-viral composite hydrogel in industrial scale by a cost-effective approach. The mechanical properties of the fabricated hydrogel could be tuned by varying the composition of MA-PMCA-PEG and GEL-MEA in the hydrogel scaffold.

*Examples:*

The present disclosure with reference to the accompanying examples describes the present invention. It is understood that the examples are provided for the purpose of illustrating the invention only and are not intended to limit the scope of the invention in any way.

**Example 1: Synthesis of poly multi-carboxylic acid-co-polyethylene glycol (PMCA-PEG).**

In the first step, PMCA-PEG was synthesized using citric acid and PEG by melt polymerization reaction under vacuum. The molecular weight of PEG varied from 400 to 6000 g/mol, and ratio of citric acid to PEG was varied from 2 to 10. Briefly, citric acid (0.2 mol), PEG (0.02 mol) and stannous chloride di-hydrate (2 mmol) were mixed and heated to 110 - 125 °C for 1 h under vacuum (100 mm Hg). Then the reaction was carried at 140 - 150 °C under high vacuum (25 mm Hg) for 5 h. After cooling to room temperature, the polymer was dissolved in 100 mL of dimethyl formamide (DMF) at 70 - 90 °C.

For characterization, 10 mL of this polymer solution was withdrawn and 60 mL of diethyl ether was added to precipitate the polymer and washed three times with fresh diethyl ether. The traces of DMF and diethyl ether present in the polymer product were removed on a vacuum oven at 90 °C for overnight. The PMCA-PEG was characterized by FTIR, $^{1}$H-NMR, and $^{13}$C-NMR, spectra (Figure. 5 - 7). Yield of the product was about 70 - 80 %.

**Example 2: Methacrylation of PMCA-PEG**

PMCA-PEG was methacrylated using glycidyl methacrylate (GMA). Briefly, to the PMCA-PEG polymer in DMF synthesized in Example 1, GMA (0.1 mol) and triethylamine (TEA) (-5 mL) were added. Then the reaction was carried at 60 - 80 °C for 18 h under dark and inert atmosphere with strong agitation. Finally, the MA-PMCA-PEG was recovered by

adding excess diethyl ether (1 - 1.5 Lit), washed for three times with diethyl ether (300 - 500 mL per washing). Then traces of DMF and diethyl ether present in the polymer product were removed by rotary evaporation at 80 - 95 °C (Figure 1). Yield of the product was about 70 %. The MA-PMCA-PEG polymer was characterized by FTIR, $^{1}H$-NMR and $^{13}C$-NMR spectral techniques (Figure. 5 - 7).

**Example 3: Maleation of gelatin**

Gelatin was dissolved in de-ionized distilled water (DDW) (10 %, w/v) and mixed with maleic anhydride dissolved in acetone (50 %, w/v). Then, the reaction was carried out at 50 °C under nitrogen atmosphere for 4 h. After the reaction period, the GEL-MEA polymer was recovered by adding excess acetone (10 times more volumes), washed with acetone for three times and dried under vacuum for overnight (Figure 2). The initial concentration of gelatin was not limited to 10 % w/v, but the concentration was varied from 4 - 15 %. Similarly, the initial concentration of maleic anhydride in acetone was not limited to 50 % w/v, but the initial concentration of maleic anhydride was varied from 25 - 75 %. Yield of the product was about 75 %. The maleation of gelatin was confirmed by $^{1}H$-NMR and $^{13}C$-NMR techniques (Figure 8 and 9).

**Example 4: Characterization of polymers**

To record NMR spectral analyses, 50 - 100 mg of the polymer samples were dissolved in 0.6 mL of $D_2O$. The $^{1}H$-NMR and $^{13}C$-NMR spectra of the polymer samples were recorded on a JEOL (ECA 500) NMR spectrometer operating at 500 MHz and 125.7 MHz for recording $^{1}H$-NMR and $^{13}C$-NMR spectra respectively. To record FTIR spectra, 2-3 mg of polymer samples were used to make KBr pellets and the FTIR spectra were recorded on an Agilent (Carry 600) FTIR spectrometer. The differential scanning calorimetric (DSC) analyses were carried on Perkin-Elmer (Pyris) differential scanning calorimeter. The PMCA-PEG and MA-PMCA-PEG samples exhibit -13.7 °C and -20.3 °C of glass transition temperature (Tg) respectively (Figure 10).

**Example 5: Process for scaling up of MA-PMCA-PEG cross-linkable polymer**

The MA-PMCA-PEG cross-linkable polymer was scaled up in a two stage reaction processes. In the first stage, the polymerization was carried in a custom designed 2 Lit glass reactor connected with (i) a vacuum line with liquid nitrogen moisture trap, (ii) thermal sensor, (iii) over head mechanical stirrer, and (iv) thermostat oil circulation. Initially, the citric acid anhydride (1 Kg), PEG (315 g) and stannous chloride dihydrate (10 g) were transferred to the 2 Lit glass reactor. Then the reactor was heated to 125 °C at 100 mm Hg vacuum for 2 h with strong agitation. The polymerization was carried at 140 - 150 °C under high vacuum (25 mm Hg) for 4 h with agitation. Then, the reaction was cooled to room temperature, and the polymer product was dissolved in DMF (0.9 Lit) at 70 - 90 °C. The vacuum line was removed from the reactor and nitrogen gas line was connected with the glass reactor via a ground joint stopper with stop cock. The entire glass reactor was covered with an aluminium foil. Triethylamine (140mL) and glycidyl methacrylate (172.8 mL) were added. Nitrogen gas was purged for 15 min and the reaction was carried at 70 °C for 15-24h under nitrogen and dark atmosphere.

In the second stage, the polymer product in DMF was transferred to a 20 Lit glass reactor connected with (i) over head mechanical stirrer, and (ii) solvent condenser. Diethyl ether as a non-solvent (10 Lit) was added and agitated strongly for 1 h to wash out the unwanted impurities. Then, the solution was kept aside for 30 min, and the supernatant solution was drained via a siphon pump. The washing process was repeated for 3 times by adding 6 Lit of diethyl ether for 3 times. After draining the supernatant, the precipitate obtained was collected in a 3 Lit RB flask and the remaining solvents were removed by rotary evaporation at 95 °C. Finally, the obtained MA-PMCA-PEG was stored under dark and cool condition (Figure 3). (Yield: 80 %)

**Example 6: Fabrication of hydrogel scaffold**

To fabricate hydrogel scaffold, the cross linkable polymers were cross-linked with an initiator in the presence of tetramethylethylenediamine (TEMED). The MA-PMCA-PEG (50 %, w/v) and GEL-MEA (50 %, w/v), ammonium persulfate (APS) (10 %, w/v) were prepared in deaerated water. The predetermined volumes (300 - 900 $\mu$L) of MA-PMCA-PEG and GEL-MEA were mixed together to get different hydrogels with various concentration of GEL-MEA and MA-PMCA-PEG (Table 1). Then, 60 $\mu$L of TEMED was added to the mixture of polymer solutions, followed by 45 $\mu$L of APS was added and incubated at 30-40 °C. After 10 - 30 min, formation of hydrogel was observed (Figure 4). The concentration of MA-PMCA-PEG and GEL-MEA was not restricted to 50 %, but it varied from 40 - 60 % w/v.

**Example 7: Determination of swelling factor of hydrogels**

To determine the swelling factor, the fabricated hydrogels were dried in a hot air oven at 40 °C overnight. After

drying, the cross-linked polymer scaffold was immersed in 5 mL of phosphate buffer (pH 7.4) and incubated at 25 °C for 4 h. Then, weight of the swelled gels was measured after carefully taking from phosphate buffer. The swelling factor of the hydrogels was calculated by using the following formula,

$$\text{Swelling factor} = (M_t - M_i)/M_i$$

Where, $M_i$ denotes initial weight of dried polymer before swelling, and $M_t$ denotes weight of swelled gel.

The gelatin based 3D hydrogel with 0.5 and 0.33 ratio of MA-PMCA-PEG/GEL-MEA exhibit swelling factor of 8.0 and 6.2 respectively (Table 1).

**Example 8: Compression modulus of hydrogels**

To measure the compression modulus, different composition of hydrogel discs (Table 1) with a diameter of 8 mm and a height of 3 - 3.5 mm were fabricated using a polypropylene mold tool. Stress-Strain measurements were carried on a TA ARES-G2 Rheometer equipped with a 2.0 Kg load cell. The hydrogel discs were compressed at a rate of 0.005 mm/s using a probe diameter of 40 mm. Stress-strain curves were plotted and compressive modulus of different hydrogel discs was determined as the slope of linear regression in the range of 3 to 15 % of strain (Figure 11 and Table 1). The compressive modulus of 3D composite hydrogel increases with increasing amount of MA-PMCA-PEG in the hydrogel scaffold. The compression modulus of the 3D hydrogel with 0.5 and 0.33 ratio of MA-PMCA-PEG/GEL-MEA is 35.1 and 19.3 kPa respectively (Table 1).

**Table 1.** Fabrication and properties of hydrogel matrices with different composition of MA-PMCA-PEG and GEL-MEA.

| Sl. No | Hydrogel type | Ratio of MA-PMCA-PEG/GEL-MEA | Swelling factor | Compression modulus (kPa) |
|---|---|---|---|---|
| 1 | HG 1 | 0.5 | 8.0 | 35.1 |
| 2 | HG 2 | 0.33 | 6.2 | 19.3 |
| 3 | HG 3 | 0.0 | 7.9 | 7.6 |

**Claims**

1. A biodegradable 3D composite hydrogel scaffold comprising:

   (i) a synthetic biodegradable polymer methacrylated poly multi-carboxylic acid-polyethylene glycol (MA-PMCA-PEG) and
   (ii) a modified natural polymer maleated gelatin (GEL-MEA).

2. The biodegradable 3D composite hydrogel scaffold as claimed in claim 1, wherein a ratio of MA-PMCA-PEG/GEL-MEA varies in a range of 0.33 to 0.5.

3. A process for fabricating a biodegradable 3D composite hydrogel scaffold comprising:

   (i) mixing of predetermined volume of a solution of MA-PMCA-PEG and a solution of GEL-MEA to obtain a polymer solution, wherein different hydrogels are obtained by varying the concentration ratio of MA-PMCA-PEG/GEL-MEA; and
   (ii) preparing hydrogel from the polymer solution and molding the hydrogel to obtain the biodegradable 3D composite hydrogel scaffold.

4. The process as claimed in claim 3, wherein the concentration of MA-PMCA-PEG is in the range of 40-60 %, w/v, and GEL-MEA is in the range of 40-60 %, w/v.

5. The process as claimed in claim 3, wherein the composite 3D hydrogel with 0.5, and 0.33 ratio of MA-PMCA-PEG/GEL-MEA exhibit swelling factor of 8.0, and 6.2 respectively, wherein the swelling factor is measured as indicated in the description.

6. The process as claimed in claim 3, wherein the compression modulus of the composite 3D hydrogel with 0.5 and 0.33

ratio of MA-PMCA-PEG/GEL-MEA is 35.1 and 19.3 kPa respectively, wherein the compression modulus is measured as indicated in the description.

**7.** A process for preparation of methacrylated poly multi-carboxylic acid-polyethylene glycol (MA-PMCA-PEG) comprising reacting poly multi-carboxylic acid-polyethylene glycol (PMCA-PEG) polymer in dimethylformamide (DMF) with glycidyl methacrylate (GMA) in the presence of triethylamine (TEA) and adding diethyl ether to recover MA-PMCA-PEG; wherein the amount of GMA is in the range of 0.02-0.1 mol, and the amount of triethylamine is in the range of 5-15 mL.

**8.** The process for preparation of MA-PMCA-PEG as claimed in claim 7, wherein the reaction is carried out at a temperature in the range of 60-80 °C for 15 to 24 h under dark and inert atmosphere with strong agitation.

**Patentansprüche**

**1.** Bioabbaubares 3D-Verbundhydrogelgerüst, umfassend:

(i) ein synthetisches bioabbaubaes Polymer, Methacrylat-Poly-Multicarbonsäure-Polyethylenglykol (MA-PMCA-PEG) und
(ii) ein modifiziertes natürliches Polymer, maleiertes Gelatine (GEL-MEA).

**2.** Bioabbaubares 3D-Verbundhydrogelgerüst nach Anspruch 1, wobei das Verhältnis von MA-PMCA-PEG/GEL-MEA in einem Bereich von 0,33 bis 0,5 variiert.

**3.** Prozess zur Herstellung eines bioabbaubaren 3D-Verbundhydrogelgerüsts, umfassend:

(i) Mischen eines vorbestimmten Volumens einer Lösung von MA-PMCA-PEG und einer Lösung von GEL-MEA, um eine Polymerlösung zu erhalten, wobei durch Variieren des Konzentrationsverhältnisses von MA-PMCA-PEG/GEL-MEA verschiedene Hydrogele erlangt werden; und
(ii) Zubereiten eines Hydrogels aus der Polymerlösung und Formen des Hydrogels, um das bioabbaubare 3D-Verbundhydrogelgerüst zu erlangen.

**4.** Prozess nach Anspruch 3, wobei die Konzentration von MA-PMCA-PEG im Bereich von 40 bis 60 Gew.-/Vol.-% und die von GEL-MEA im Bereich von 40 bis 60 Gew.-/Vol.-% liegt.

**5.** Prozess nach Anspruch 3, wobei das 3D-Verbundhydrogel mit einem Verhältnis von MA-PMCA-PEG/GEL-MEA von 0,5 und 0,33 einen Quellfaktor von 8,0 beziehungsweise 6,2 aufweist, wobei der Quellfaktor wie in der Beschreibung angegeben gemessen wird.

**6.** Prozess nach Anspruch 3, wobei der Kompressionsmodul des 3D-Verbundhydrogels mit einem Verhältnis von MA-PMCA-PEG/GEL-MEA von 0,5 und 0,33 bei 35,1 beziehungsweise 19,3 kPa liegt, wobei der Kompressionsmodul wie in der Beschreibung angegeben gemessen wird.

**7.** Prozess zur Vorbereitung von methacryliertem Poly-Multicarbonsäure-Polyethylenglykol (MA-PMCA-PEG), umfassend die Reaktion des Polymers Poly-Multicarbonsäure-Polyethylenglykol (PMCA-PEG) in Dimethylformamid (DMF) mit Glycidylmethacrylat (GMA) in Gegenwart von Triethylamin (TEA) und die Zugabe von Diethylether, um MA-PMCA-PEG zu gewinnen; wobei die Menge an GMA im Bereich von 0,02 bis 0,1 mol liegt und die Menge an Triethylamin im Bereich von 5 bis 15 ml liegt.

**8.** Prozess zur Vorbereitung von MA-PMCA-PEG nach Anspruch 7, wobei die Reaktion bei einer Temperatur im Bereich von 60 bis 80 °C für 15 bis 24 Stunden in dunkler und inerter Umgebung mit starkem Rühren durchgeführt wird.

**Revendications**

**1.** Échafaudage d’hydrogel composite 3D biodégradable comprenant :

(i) un polymère synthétique biodégradable en acide polycarboxylique méthacrylépolyéthylène glycol (MA-PMCA-PEG) et
(ii) un polymère naturel modifié en gélatine maléatée (GEL-MEA).

2. Échafaudage d'hydrogel composite 3D biodégradable selon la revendication 1, dans lequel un rapport MA-PMCA-PEG/GEL-MEA varie entre 0,33 et 0,5.

3. Procédé de fabrication d'un échafaudage d'hydrogel composite 3D biodégradable comprenant : (i) le mélange d'un volume prédéterminé d'une solution de MA-PMCA-PEG et d'une solution de GEL-MEA pour obtenir une solution polymère, dans lequel les différents hydrogels étant obtenus en faisant varier le rapport de concentration de MA-PMCA-PEG/GEL-MEA ; et (ii) la préparation d'un hydrogel à partir de la solution polymère et le moulage de l'hydrogel pour obtenir l'échafaudage d'hydrogel composite 3D biodégradable.

4. Procédé selon la revendication 3, dans lequel la concentration de MA-PMCA-PEG est comprise entre 40 et 60 % (masse/vol), et celle de GEL-MEA est entre 40 et 60 % (masse/vol).

5. Procédé selon la revendication 3, dans lequel l'hydrogel composite 3D, présentant un rapport MA-PMCA-PEG/GEL-MEA de 0,5 et 0,33, présente un facteur de gonflement de 8,0 et 6,2 respectivement, dans lequel, le facteur de gonflement est mesuré comme indiqué dans la description

6. Procédé selon la revendication 3, dans lequel le module de compression de l'hydrogel composite 3D avec un rapport MA-PMCA-PEG/GEL-MEA de 0,5 et 0,33 est respectivement de 35,1 et 19,3 kPa, dans lequel le module de compression est mesuré comme indiqué dans la description

7. Procédé de préparation de poly(acide multi-carboxylique-polyéthylène glycol) méthacrylaté (MA-PMCA-PEG) comprenant la réaction du polymère poly(acide multi-carboxylique-polyéthylène glycol) (PMCA-PEG) dans le diméthylformamide (DMF) avec du méthacrylate de glycidyle (GMA) en présence de triéthylamine (TEA) et l'ajout d'éther diéthylique pour récupérer le MA-PMCA-PEG, dans lequel la quantité de GMA est comprise entre 0,02 et 0,1 mol, et la quantité de triéthylamine est comprise entre 5 et 15 ml.

8. Procédé de préparation du MA-PMCA-PEG selon la revendication 7, dans lequel la réaction est effectuée à une température comprise entre 60 et 80 °C pendant 15 à 24 h sous atmosphère sombre et inerte avec une forte agitation.

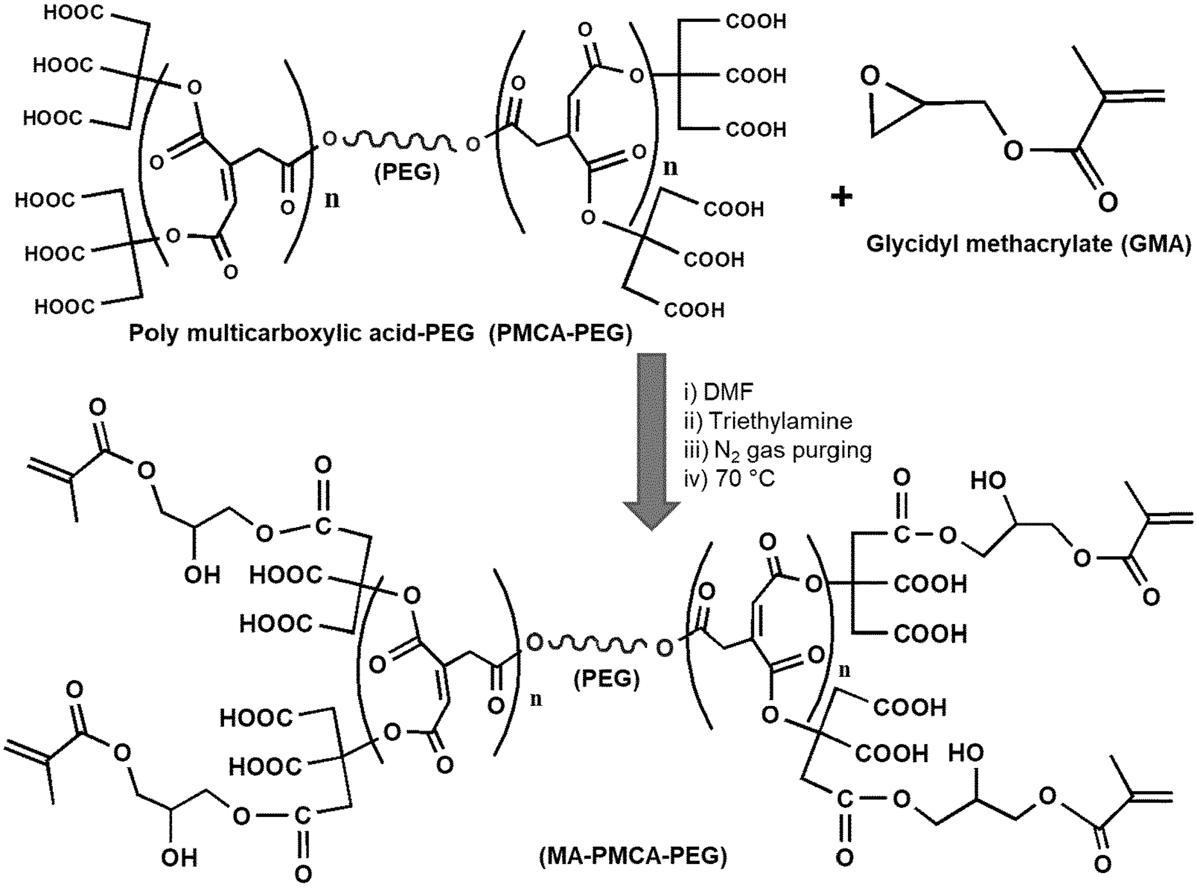


Poly multicarboxylic acid-PEG (PMCA-PEG)

Glycidyl methacrylate (GMA)

(MA-PMCA-PEG)

Figure 1

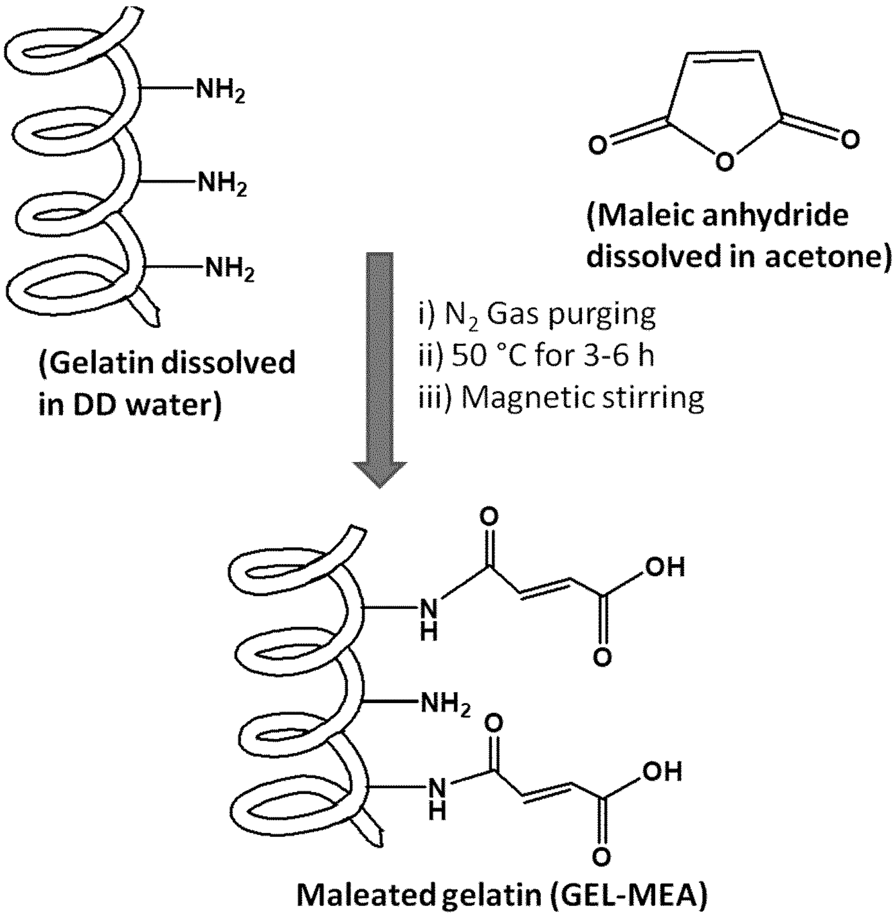
(Gelatin dissolved in DD water)
(Maleic anhydride dissolved in acetone)
i) $N_2$ Gas purging
ii) 50 °C for 3-6 h
iii) Magnetic stirring
Maleated gelatin (GEL-MEA)

Figure 2

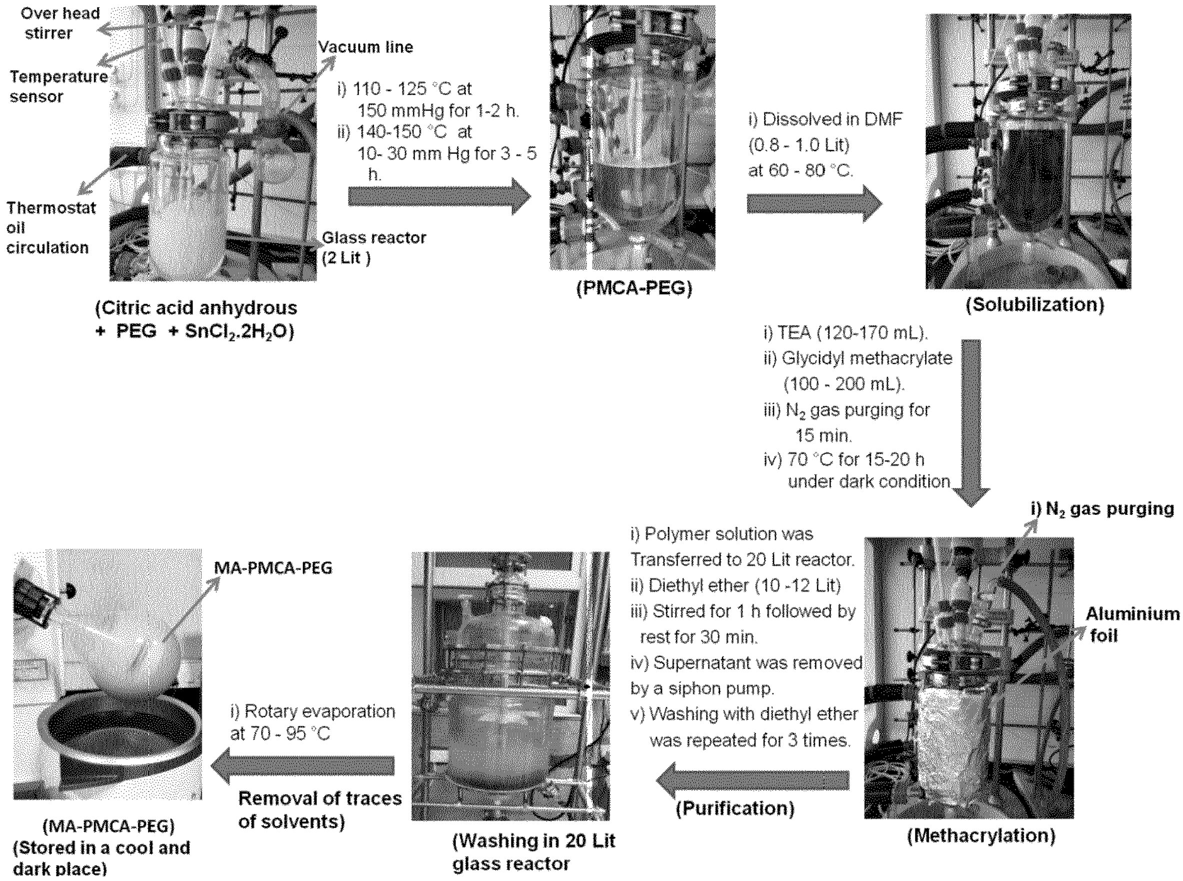


**Figure 3**

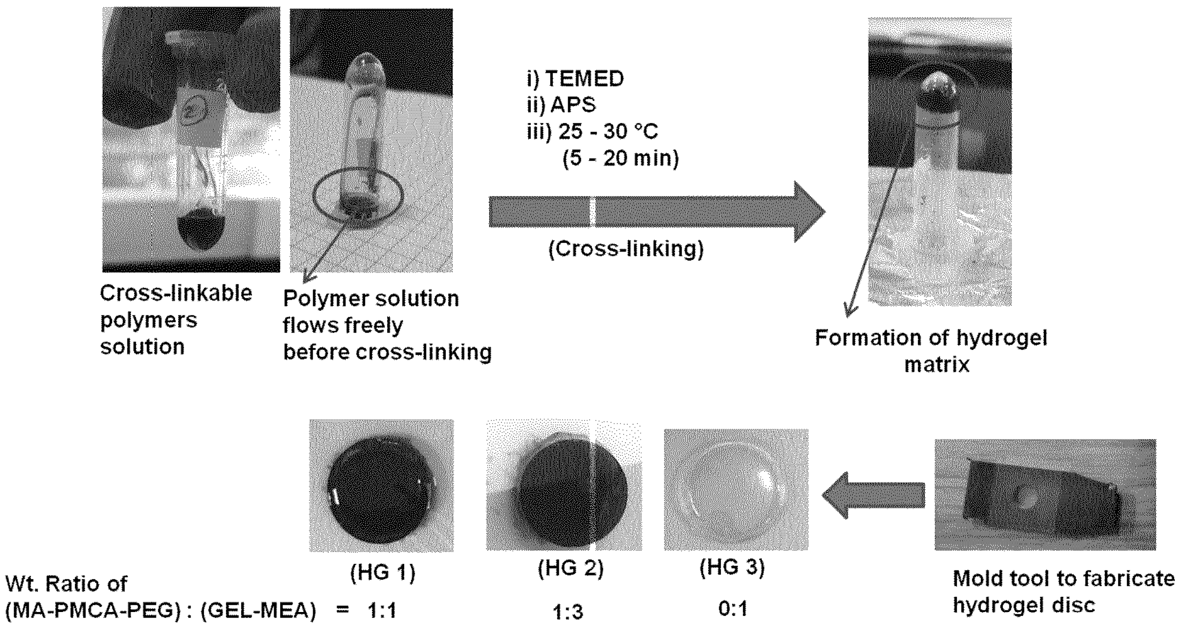


**Figure 4**

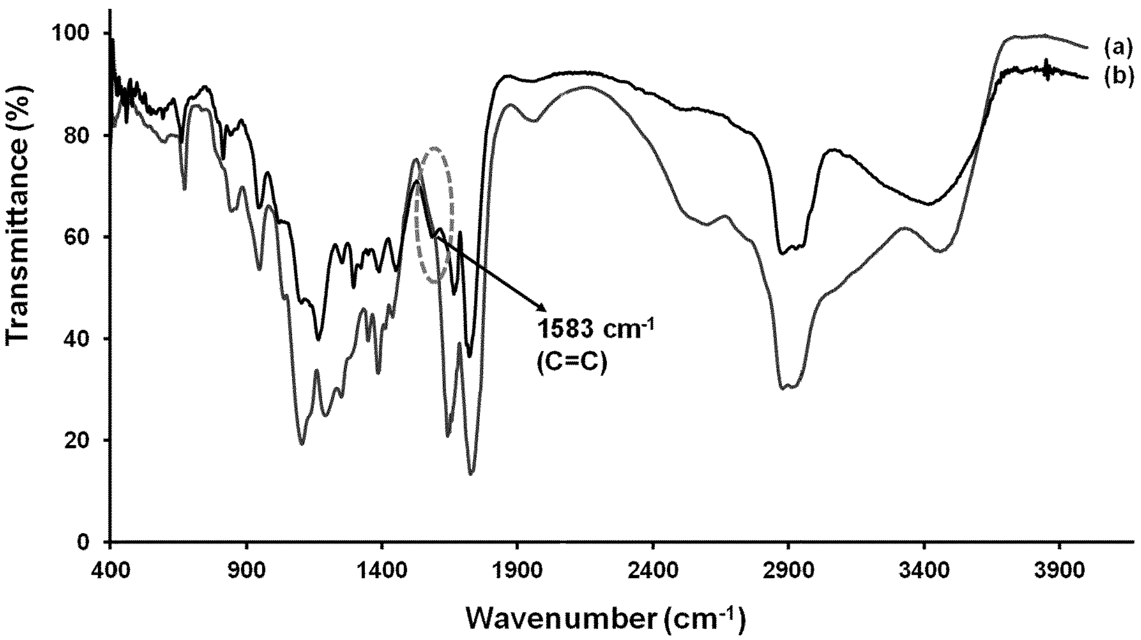

100
80
60
40
20
0
Transmittance (%)
400
900
1400
1900
2400
2900
3400
3900
Wavenumber (cm-1)
(a)
(b)
1583 cm-1
(C=C)


Figure 5

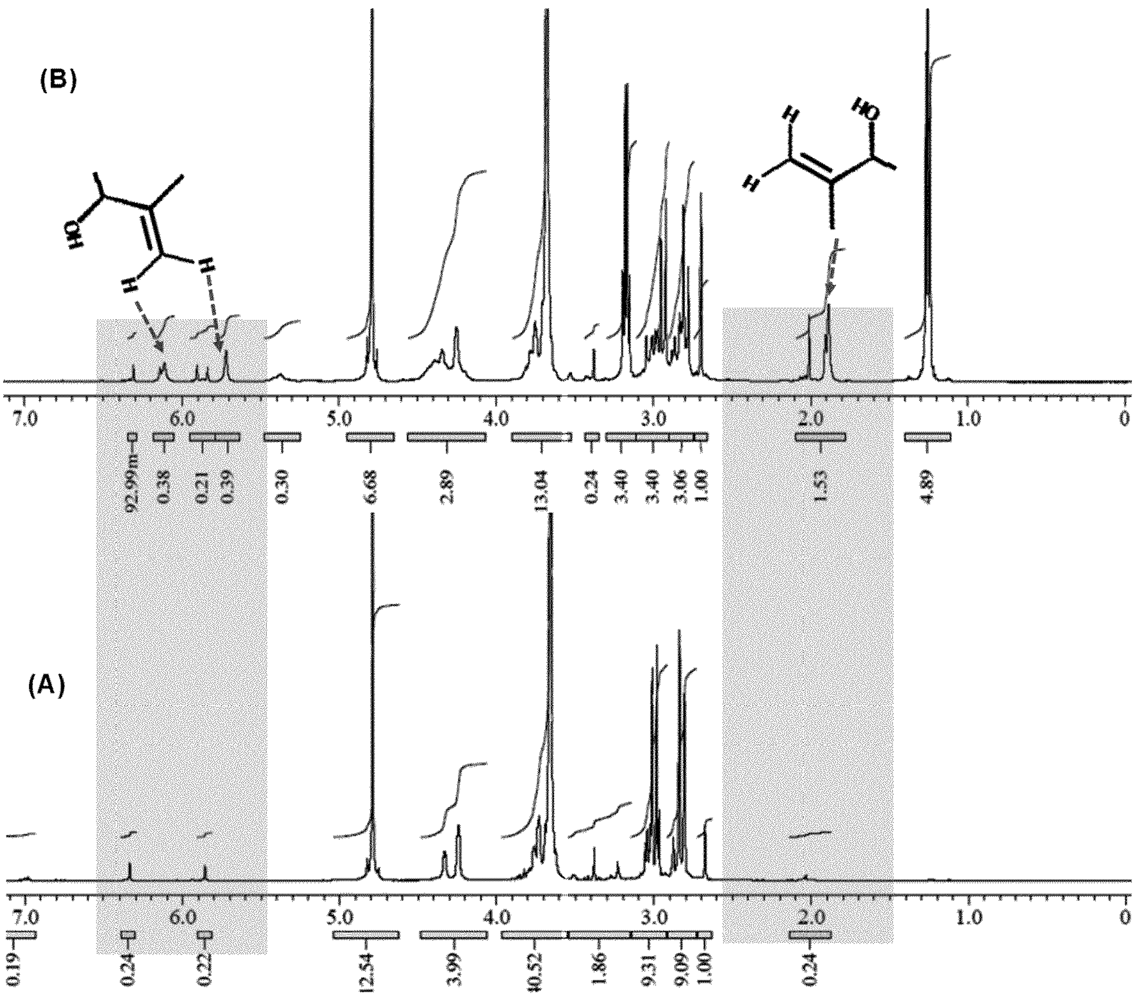
(B)
(A)
7.0
6.0
5.0
4.0
3.0
2.0
1.0
0
92.99m
0.38
0.21
0.39
0.30
6.68
2.89
13.04
0.24
3.40
3.40
3.06
1.00
1.53
4.89
0.19
0.24
0.22
12.54
3.99
10.52
1.86
9.31
9.09
1.00
0.24

**Figure 6**

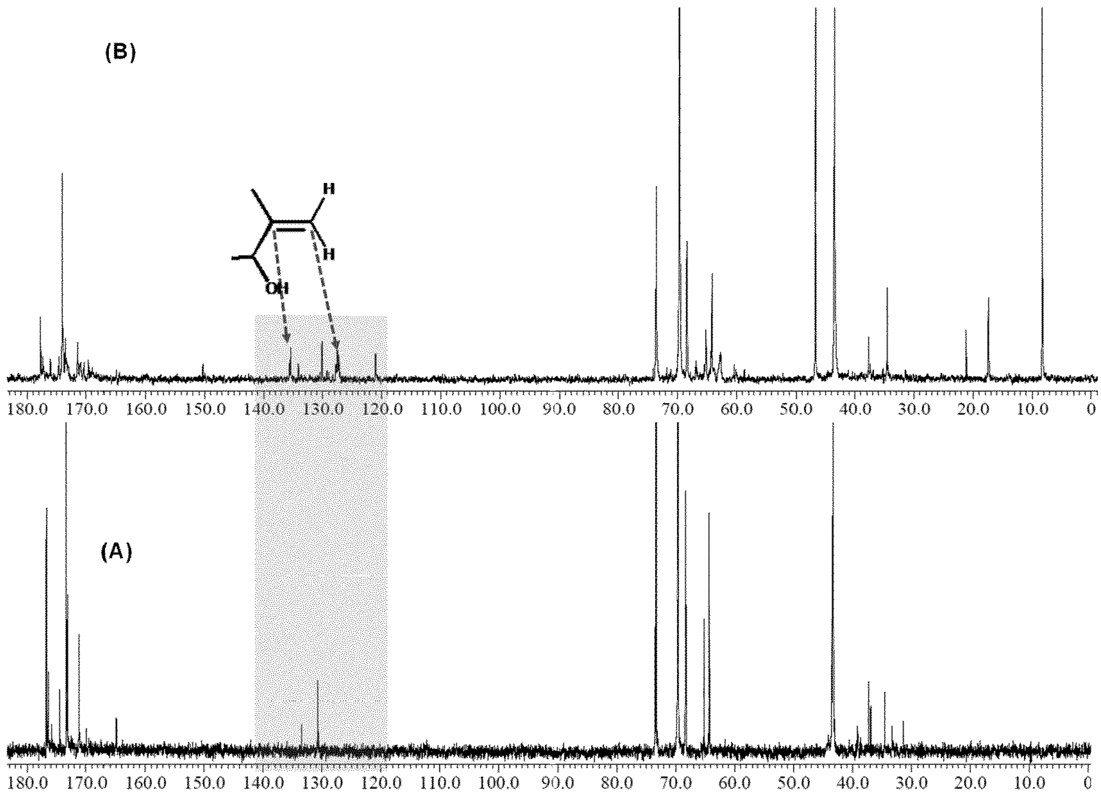
(B)
180.0 170.0 160.0 150.0 140.0 130.0 120.0 110.0 100.0 90.0 80.0 70.0 60.0 50.0 40.0 30.0 20.0 10.0 0
(A)
180.0 170.0 160.0 150.0 140.0 130.0 120.0 110.0 100.0 90.0 80.0 70.0 60.0 50.0 40.0 30.0 20.0 10.0 0

**Figure 7**

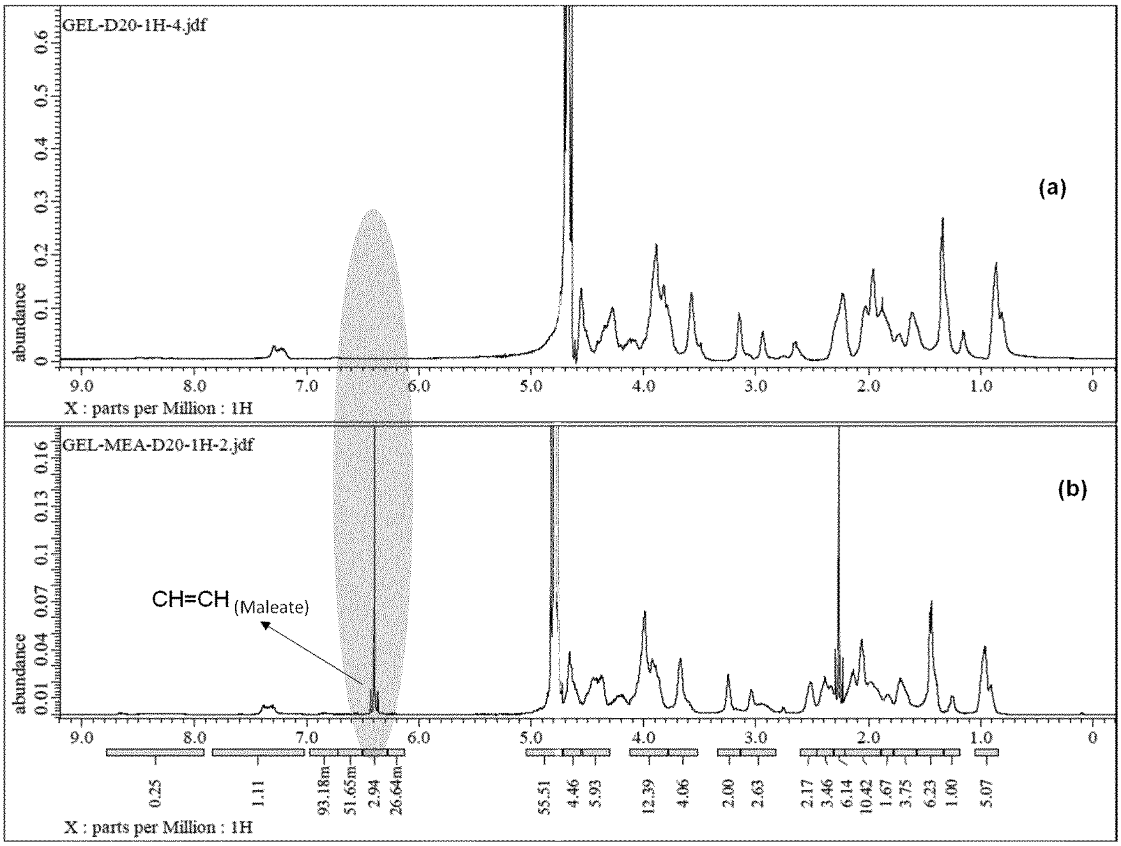
GEL-D20-1H-4.jdf
(a)
abundance
X : parts per Million : 1H
GEL-MEA-D20-1H-2.jdf
(b)
CH=CH (Maleate)
abundance
X : parts per Million : 1H

**Figure 8**

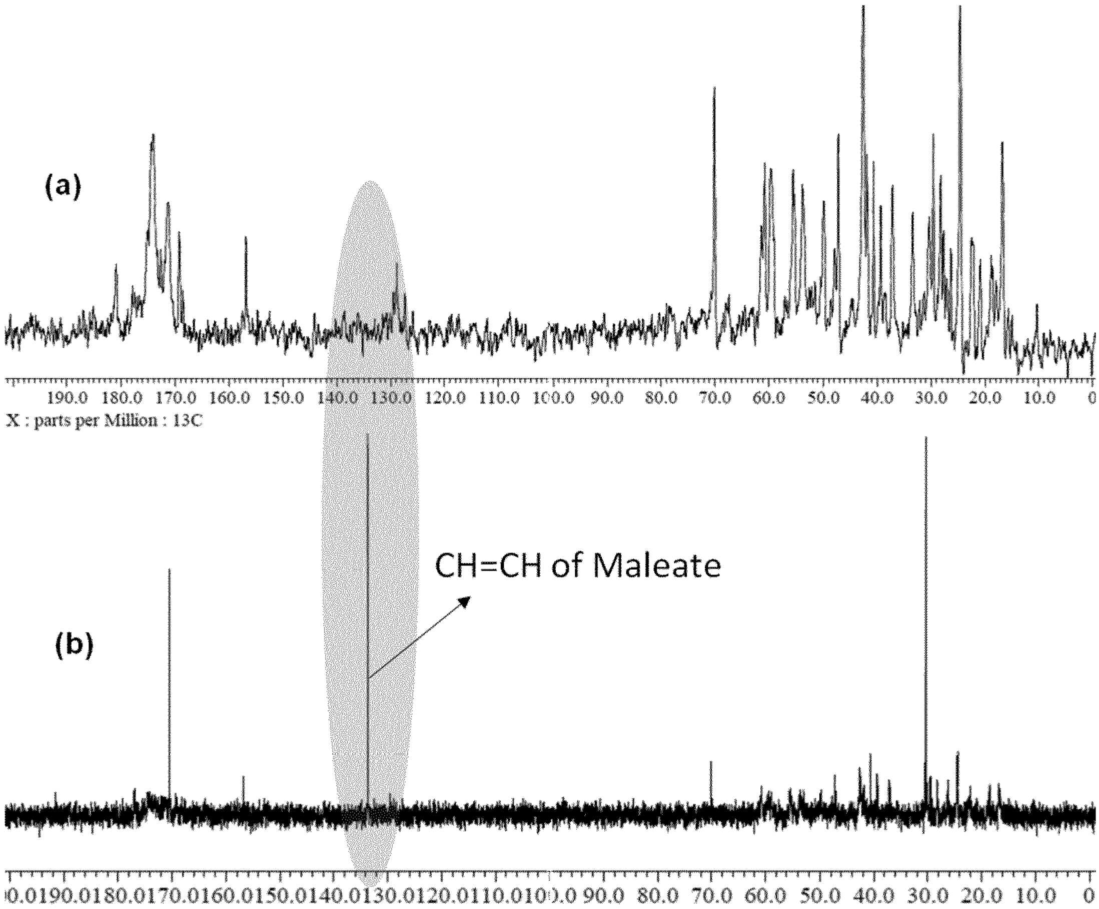


Figure 9

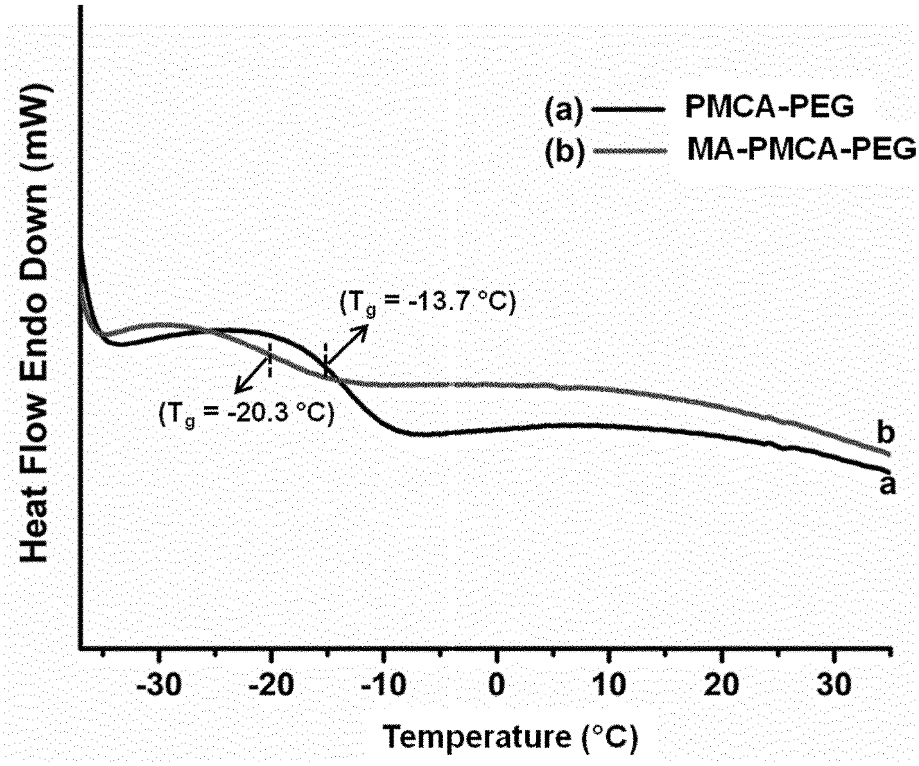


Figure 10

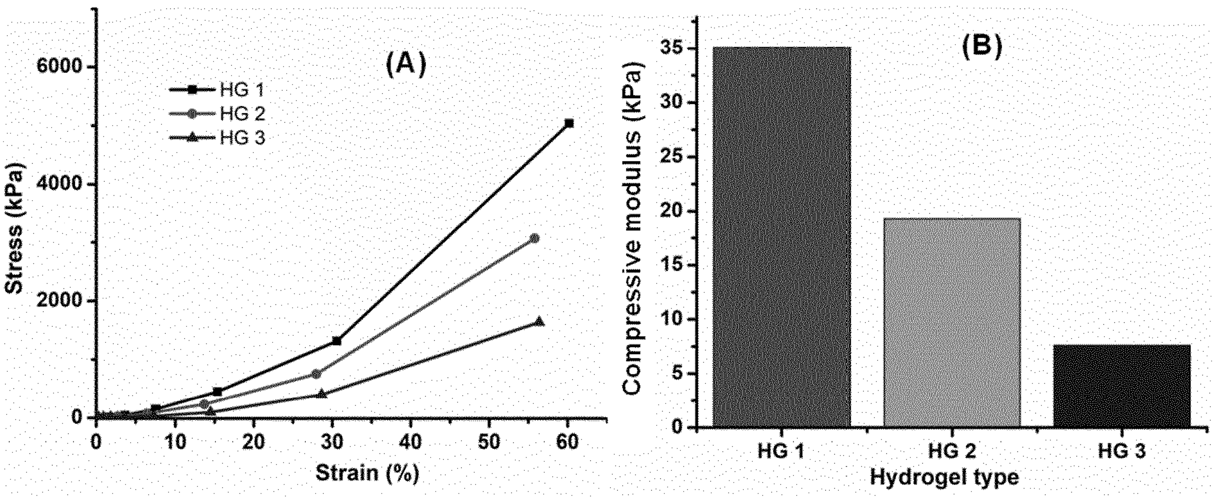


Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006004951 A2
- US 7670616 B2
- WO 2019226120 A1
- EP 2448610 B1
- US 5834232 A
- US 20140315805 A1
- US 20141023 B
- CN 108102112
- US 2021346576 A


### Non-patent literature cited in the description


- **NAEINI et al.** PCA)-block-poly(ethylene glycol) (PEG) copolymer. *Nanomedicine: Nanotechnology, Biology, and Medicine*, 2010, vol. 6, 556-562
- **NAEINI et al.** PCA-PEG-PCA polymer to synthesize necklace shaped gold NPs. *European Polymer Journal*, 2010, vol. 46, 165-170
- **GAJENDIRAN et al.** *Scientific Reports*, 2017, vol. 7 (16418)
- *Journal Material Chemistry B*, 2014, vol. 2, 418-427
- **KIM et al.** *ACS Appl. Bio Mater.*, 2020, vol. 3, 1646-1655
- **BULCKE et al.** *Biomacromolecules*, 2000, vol. 1, 31-38
- **MONUZ et al.** *Biomater. Sci.*, 2014, vol. 2, 1063-1072
- **SUNGJUN et al.** *Tissue Eng. Regen. Med.*, 2022, vol. 19, 309-319
- **SUKHLAAIED et al.** *Polym. Bull.*, 2016, vol. 8
- *Chemistry and Materials Research.*, 2015, vol. 7 (5)